# EUROPEAN PATENT APPLICATION

(11) **EP 4 789 668 A1**
(43) Date of publication of application: **12.08.2026**
(21) Application number: 25382112.8
(22) Date of filing: 11.02.2025
(51) Int. Cl.: A61K 31/472, A61P 19/08, A61P 19/10

(54) **ATRACURIUM SALT FOR USE IN THE TREATMENT OF BONE FRAGILITY-RELATED DISEASES**

(71) Applicant: Administración General de la Comunidad Autónoma de Euskadi, 01010 Vitoria-Gasteiz (Álava) (ES)
(72) Inventor: Rodriguez López, Clara Isabel, 01010 Vitoria-Gasteiz (ES); Infante Martínez, Arantza, 01010 Vitoria-Gasteiz (ES); Gener Querol, Blanca, 01010 Vitoria-Gasteiz (ES); Alcorta Sevillano, Natividad, 01010 Vitoria-Gasteiz (ES)
(74) Representative: Pons IP

(57) **Abstract**

The invention relates to the atracurium salt, preferably cisatracurium besylate, or composition comprising it, for use in the treatment and/or prevention of a bone fragility-related disease in a subject, preferably osteogenesis imperfecta or osteoporosis.

## Description

The invention belongs to the technical field of the medicine, particularly of the bone disease. The invention relates to the atracurium salt, preferably cisatracurium besylate, for use in the treatment and/or prevention of bone fragility-related diseases in a subject, particularly in the treatment and/or prevention of osteogenesis imperfecta or osteoporosis.

### BACKGROUND ART

Osteogenesis Imperfecta (OI), is a rare genetic disorder that encompasses a diverse group of conditions affecting the connective tissue. It is also referred to as "brittle bone disease", since it is mainly characterized by bone fragility and fragility fracture (FF) due to decreased Bone Mineral Density (BMD) (Marini, J. C., et al., 2017, Osteogenesis imperfecta. Nat Rev Dis Primers, 3, 17052)*.*

It is a very heterogeneous disease, where patients present a wide range of phenotypes, from mild to very severe forms characterised by multiple fractures and major bone deformities to even lethal, and mutations have been identified in at least 22 genes associated with this disease. The pathogenesis is associated with alterations in the type I collagen molecule (COL1), the main protein component of the bone extracellular matrix (ECM) whose function is to give strength and flexibility to the bone. Approximately 85% of OI cases are caused by mutations in the COL1A1 or COL1A2 genes, which encode the α1 and α2 chains of COL1. However, mutations in up to 22 other genes have been identified in patients with OI, highlighting the genetic heterogeneity of this disorder. These genes are associated with the synthesis, processing (BMP), post-translational modifications (CRTAP, LEPRE1, PPIB, TEMEM38B), folding and trafficking (SERPINH1, KDELR2, FKBP10, PLOD2) of COL1 (Valencia, M., et al., 2014, Report of a newly identified patient with mutations in BMP1 and underlying pathogenetic aspects. Am J Med Genet A, 164A (5), 1143-1150*).*

OI is primarily characterized by bone fragility and osteopenia, which result in a high incidence of fractures in atypical locations. These fractures may occur with minimal or no trauma. For example, some patients with severe OI present prenatal fractures and shortening of long bones in utero. In addition, bone deformities, including bowing of long bones, scoliosis and rib cage deformities, and short stature have been observed in some patients with OI (Castelein, R. M., et al., 2019, Complex spine deformities in young patients with severe osteogenesis imperfecta: current concepts review. J Child Orthop, 13 (1), 22-32*)*.

Nowadays there is no cure for OI (Botor, M., et al., 2021, Osteogenesis Imperfecta: Current and Prospective Therapies. Biomolecules, 11 (10*)),* existing treatments only attempt to improve symptoms and have limited efficacy, and the majority of the available treatments have been extrapolated from other bone fragility-related diseases, such as osteoporosis. Therefore, these treatments are restricted in their scope and efficacy, due to the genetic and clinical heterogeneity of OI. The principal goals of the treatment are to enhance bone density and strength, mitigate fracture risk, alleviate pain, enhance mobility and functional independence, and forestall long-term complications. Treatments employed for patients with OI can be broadly classified into two principal groups: orthopedic management and pharmacological therapy.

The current treatment of OI is dependent upon the age, type, severity, and functional status of the patient. Thus, individuals with mild OI may only require monitoring for complications, whereas those with more severe forms will typically need multidisciplinary management, comprising physical therapy, occupational therapy, orthopedic interventions, pharmacological therapy and follow-up by other subspecialists (Marom, R., et al., 2020, Osteogenesis imperfecta: an update on clinical features and therapies. Eur J Endocrinol, 183 (4), R95-R106)*.*

Overall, although significant progress has been made to develop therapies for bone fragility-related diseases, particularly for OI, the development of effective therapies remains a pressing need. Most of the available treatments are not effective for all the patients or present severe adverse events, including long half-life and hipercalcemia. New methods of stimulating bone creation in affected patients have the potential to become a strategy capable of modifying the course of the bone fragility-related diseases. So, urging the development of new, more effective and personalised therapies for OI or osteoporosis.

### DESCRIPTION OF THE INVENTION

The present invention relates to the effect of an atracurium salt, particularly G04 molecule (i.e. cisatracurium besylate) in the treatment and/or prevention of bone fragility-related diseases in a subject, particularly in the treatment and/or prevention of osteogenesis imperfecta (OI) or osteoporosis. The effect of cisatracurium besylate has been studied in MSCs from OI patients *in vitro,* and in OI murine models *in vivo.*

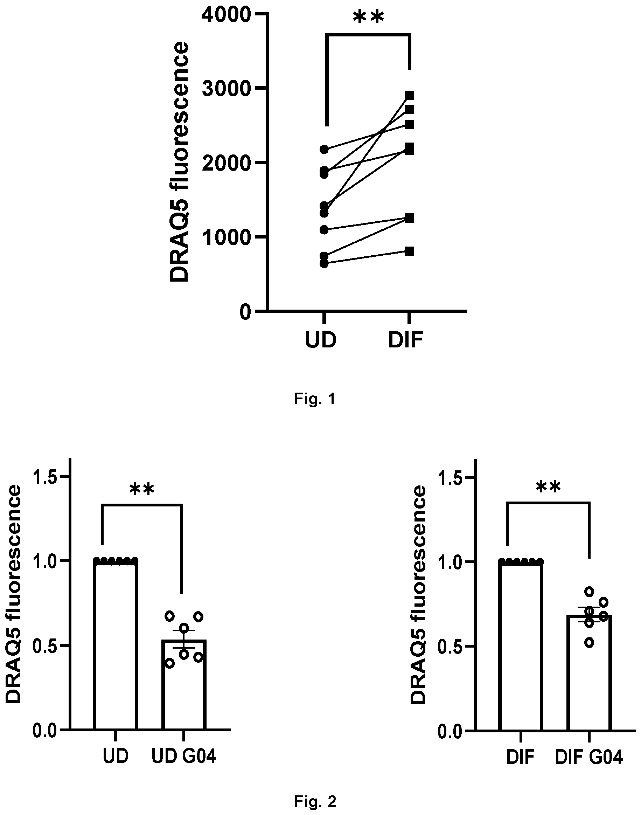

First the response to the differentiation stimuli of each OI-MSCs line was tested. An increase of the number of cells is expected under osteogenic differentiation conditions. All the DIF OI-MSCs lines studied, reflected a higher number of cells when comparing with the UD ones (Figure 1). In the OI-MSCs, a decrease on the number of cells was observed when treated with the concentration of 10 µM G04, in all the conditions studied (Figure 2).

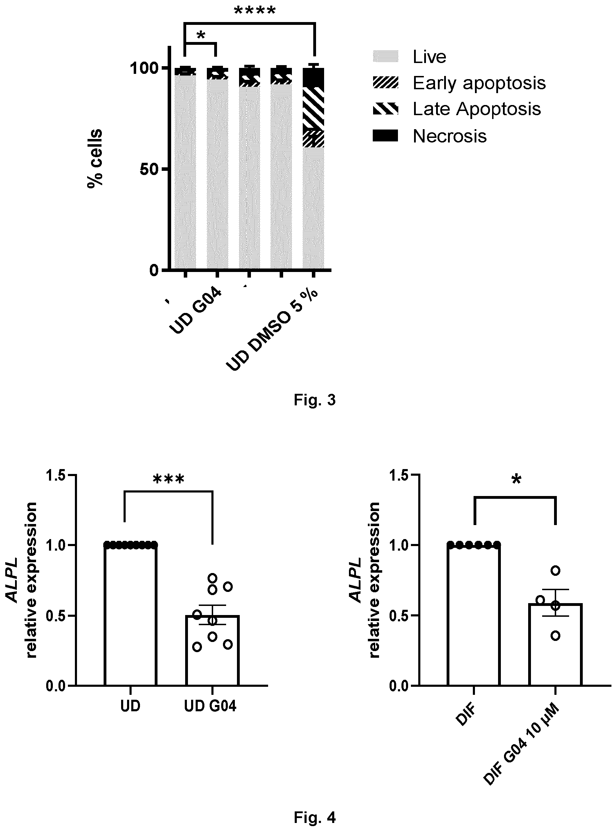

In order to study if the treatment of 10 µM G04 induces apoptosis, the OI-MSCs lines were evaluated by flow cytometry. Two different markers were used to divide live, early apoptotic, late apoptotic and death, necrotic cells. The positive control with DMSO showed increased percentages of apoptotic cells. However, the reduction in the number of OI-MSCs treated with 10 µM G04 was not due to an increase in the apoptosis of the cells. Thus, it may be due to a decreased proliferation (Figure 3).

Then, the OI-MSCs cell lines and/or replicates that showed an increased alp activity were selected. The 10 µM of G04 delays the differentiation since both the alkaline phosphatase expression and activity are decreased (Figure 4).

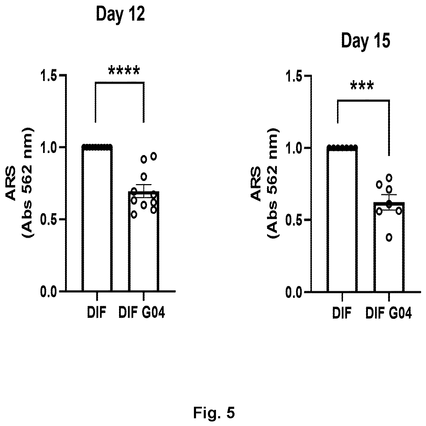

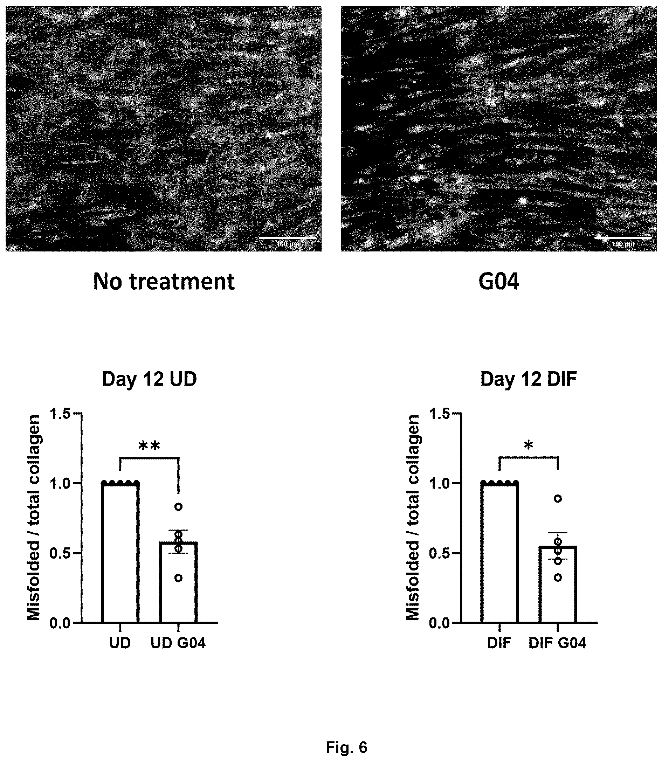

Late stages of osteogenic differentiation *in vitro* are studied, since cells must remain attached during, at least, two weeks, using the mineralization technique with alizarin red staining (ARS), which measures the calcium deposits secreted by the cells during differentiation. Under osteogenic differentiation conditions, OI-MSCs demonstrated an increase of calcium deposits (measured by ARS). However, when differentiated OI-MSCs were treated with 10 µM G04, calcium content was decreased in the cell culture, when comparing with untreated controls (Figure 5). Further, a 10 µM G04 treatment, the ratio of misfolded collagen/total collagen decreased, indicating less amount of mutant collagen after the treatment (Figure 6).

For the *in vivo* studies, OIM mice were intraperitoneally treated with 0.8 mg/kg of G04. After the treatment, right tibia was isolated and evaluated under 3 point bending test and the maximum force (Maximum Load) needed to break the bone was measured. Results demonstrated a statistically significant increase in the Maximum Load of mice treated with the molecule G04 comparing with controls (Figure 7). Regarding bone microarchitecture, tibia of G04 treated mice showed amelioration in some of the bone parameters studied (Figure 8 and 9).

Thus, a first aspect of the present invention related to a pharmaceutically acceptable atracurium salt for use in the treatment and/or prevention of bone fragility-related diseases in a subject, preferably in the treatment and/or prevention of osteogenesis imperfecta (OI) or osteoporosis in a subject, hereinafter the "use of the invention".

The atracurium is the common name of the compound 5-[3-[1-[(3,4-dimethoxyphenyl)methyl]-6,7-dimethoxy-2-methyl-3,4-dihydro-1H-isoquinolin-2-ium-2-yl]propanoyloxy]pentyl 3-[1-[(3,4-dimethoxyphenyl)methyl]-6,7-dimethoxy-2-methyl-3,4-dihydro-1H-isoquinolin-2-ium-2-yl]propanoate, and it is represented by the structural formula (I):

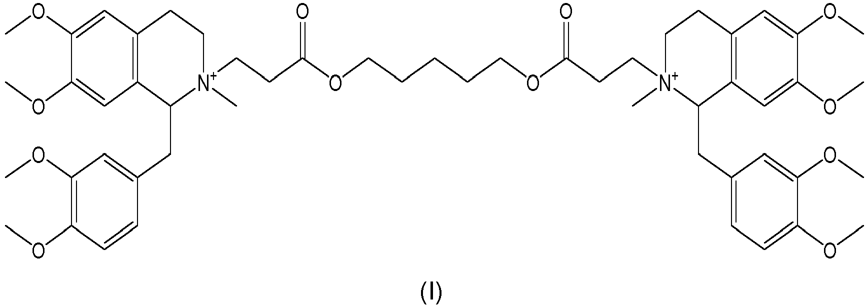

The pharmaceutically acceptable atracurium salt according to the invention including pharmaceutically acceptable anions, preferred anions including the halide, eg chloride, bromide or iodide, sulphate, phosphate, hydrogen phosphate, acetate, propionate, succinate, maleate and organosulphonate, eg methanesulphonate (mesylate), benzenesulphonate (besylate), p-toluenesulphonate (tosylate) and naphthalenesulphonate anions, the mesylate and besylate anions being especially preferred. More preferably, the salt is from besylate.

Atracurium compound has four chiral centers, which should theoretically allow for 16 possible isomers. Due to the symmetry of the molecule the number of possible isomers is reduced to 10. Cisatracurium is one of 10 isomers of atracurium.

In a preferred embodiment of the present invention, the atracurium is cisatracurium,
In a more preferred embodiment, the pharmaceutically acceptable atracurium salt is cisatracurium besylate.

The "cisatracurium besylate" of formula (represented by the structural formula (II)) is a bisbenzyltetrahydroisoquinlinium that has effect as a non-depolarizing neuromuscular-blocking drug, used adjunctively in anesthesia to facilitate endotracheal intubation and to provide skeletal muscle relaxation during surgery or mechanical ventilation, in adults and pediatric patients 1 month to 12 years of age. The chemical formula of cisatracurium besylate is C₆₅H₈₂N₂O₁₈S₂ and a IUPAC name: benzenesulfonate;5-[3-[(1R,2R)-1-[(3,4-dimethoxyphenyl)methyl]-6,7-dimethoxy-2-methyl-3,4-dihydro-1H-isoquinolin-2-ium-2-yl]propanoyloxy]pentyl 3-[(1R,2R)-1-[(3,4-dimethoxyphenyl)methyl]-6,7-dimethoxy-2-methyl-3,4-dihydro-1H-isoquinolin-2-ium-2-yl]propanoate.

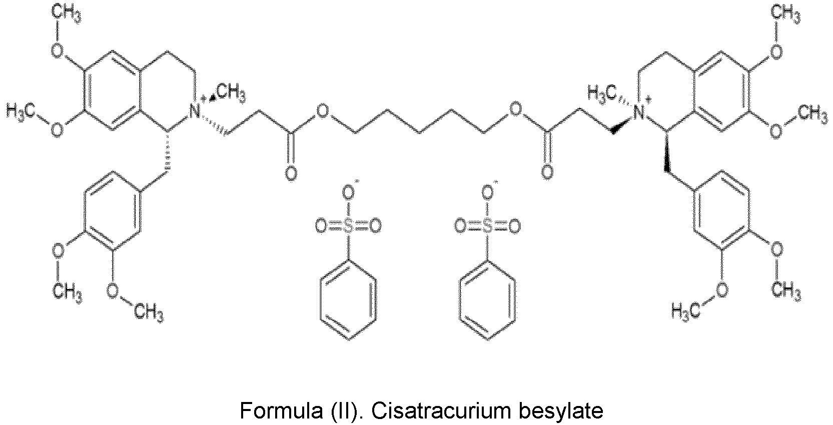

As used in the present invention, the term "to treat" or "treatment" comprise preventing and/or inhibiting the disease or disorder, i.e., stopping its development; relieving the disease or disorder, i.e., causing regression of the disease or disorder; and/or stabilizing the disease or disorder in a subject. In the present invention, the disease or disorder is a bone fragility-related disease, preferably the bone fragility-related disease is osteogenesis imperfecta (OI) or osteoporosis, or a condition caused by osteogenesis imperfecta. The "treatment" of a disease or disorder condition may also lead to a partial response (e.g., amelioration of symptoms) or complete response (e.g., disappearance of symptoms) of the subject/patient suffering from the disease, or disorder.

As used herein, the term "prevention" refers to the avoidance of occurrence of the disease or disorder in a subject, particularly when the subject has predisposition for it, but has not yet been diagnosed, or minimize or hinder the occurrence of the disease or disorder in a subject. It may also comprises reducing the risk of developing a disease or disorder in a subject.

In the present invention "subject" means any animal, preferably a mammal, more preferably a primate, in particular a human being, of any breed, sex or age. In a preferred embodiment, alone or in combination with the rest of preferred embodiments, the subject is a human.

In a preferred embodiment of the present invention, the subject is a human, preferably a human under 18 years of age.

In other preferred embodiment of the present invention, the human is a male.

According to the use of the invention, the pharmaceutically acceptable atracurium salt is used for the treatment and/or prevention of a bone fragility-related disease in a subject, preferably in the treatment and/or prevention of osteogenesis imperfecta (OI) or osteoporosis.

As used herein, the term " bone fragility-related disease" refers to diseases associated with bone fragility and changes in the material and structural properties of bone, particularly reduction in bone strength, bone mass, bone density and reduction of the connectivity of bone resulting in bone fractures. It is widely known to the person skilled in the art the methods and techniques for analyzing and assessing bone fragility in a patient and determining whether the subject has a bone fragility-related disease, as compared to a healthy subject. The techniques or methods for the study of bone structure of patients comprise image-based techniques, such as µCT and parameters as Bone Mineral Density (BMD), Cortical Bone Area (Ct.Ar), Medullary Area (Me.Ar), Cortical Thickness (Ct.Th), Trabecular Area (T.Ar), Trabecular thickness (Tb.Th), Trabecular Number (Tb.N), Trabecular Space (Tb.Sp). Examples of bone fragility-related diseases include, with limitation to osteogenesis imperfecta (OI), osteoporosis, osteoarthritis or osteomalacia.

In a preferred embodiment, the invention relates to the pharmaceutically acceptable atracurium salt for use the treatment and/or prevention of a bone fragility-related disease in a subject, wherein the bone fragility-related disease is osteogenesis imperfecta or osteoporosis.

As knows the skilled person in the art, osteogenesis imperfecta is classified into four distinct subgroups. Type I OI is regarded as the mildest form, characterized by the presence of blue sclera without any evidence of bone deformities. Type II is extremely severe and is associated with perinatal mortality. Type III is the most severe form observed in patients who survive, presenting severe progressive deformities and an extremely short stature. Finally, type IV represents an intermediate form between type I and III. These patients exhibit a moderate degree of severity, manifesting mild to moderate bone deformities, short stature, and normal sclera.

Thus, in a more preferred embodiment of the present invention, the pharmaceutically acceptable atracurium salt is used in the treatment and/or prevention of osteogenesis imperfecta, wherein the osteogenesis imperfecta is selected from the list consisting of: Type I osteogenesis imperfecta, Type II osteogenesis imperfecta, Type III osteogenesis imperfecta and Type VI osteogenesis imperfecta.

The pharmaceutically acceptable atracurium salt, more preferably the cisatracurium besylate, can be comprised in, or it can be administered, as an active compound in the form of a composition. Thus, in other aspect, the invention relates to a composition comprising the pharmaceutically acceptable atracurium salt, more preferably the cisatracurium besylate compound, hereinafter the "composition of the invention", for use in the treatment and/or prevention of a bone fragility-related disease, preferably osteogenesis imperfecta or osteoporosis, or a condition caused by the bone fragility-related disease, preferably a subject in need thereof.

The composition of the invention, may be formulated for pharmaceutical administration, i.e., forming part of pharmaceutical products to be administered to the subject by any means of administration. Thus, in a preferred embodiment, the composition of the invention is a pharmaceutical composition or preparation ("pharmaceutical composition of the invention").

The term "pharmaceutical composition" herein refers to any substance used for prevention, diagnosis, alleviation, treatment or cure of diseases in humans or animals. The pharmaceutical composition of the invention can be used either alone or in combination with other pharmaceutical compositions. The term pharmaceutical composition and drug are used in this invention interchangeably.

Likewise, the composition of the invention may comprise an excipient and/or carrier, preferably, a pharmaceutically acceptable excipient, a pharmaceutically acceptable carrier and /or an adjuvant, or any combinations thereof.

The term "excipient" refers to a substance which helps to absorb any of the components of the composition of the invention, stabilizes said components or helps in the preparation of the composition in the sense of giving it consistency or, if necessary, providing flavors which make them more pleasant. Thus, excipients could have the function of keeping the components bound together, such as for example starches, sugars or celluloses, a sweetening function, a colorant function, the function of protecting the medicament, such as for example isolating it from the air and/or moisture, a filler function for a tablet, capsule or any other form of formulation, such as for example dibasic calcium phosphate, a disintegrating function to facilitate the dissolution of the components and their absorption in the intestine, without excluding other types of excipients not mentioned in this paragraph. Therefore, the term "excipient" is defined as any material included in the galenic forms which is added to the active ingredients or to its associations to enable its preparation and stability, modify its organoleptic properties or determine the physical/chemical properties of the composition and its bioavailability. The "pharmaceutically acceptable" excipient should allow for the activity of the compounds of the pharmaceutical composition, that is to say, for it to be compatible with said components. Examples of excipients are agglutinants, fillers, disintegrators, lubricants, coaters, sweeteners, flavorings and colorants. Non-limiting, more specific examples of acceptable excipients are starches, sugars, xylitol, sorbitol, calcium phosphate, steroid fats, talc, silica or glycerin, amongst others.

The term "carrier", as used herein, refers to a substance used in the pharmaceutical composition or medicament to dilute any component of the present invention included therein to a given volume or weight. The vehicle function is to facilitate the incorporation of other elements, allow better dosage and administration or give substance and form to the composition. When the presentation is liquid, the pharmacologically acceptable carrier is the diluent. In a particular embodiment, the carrier is pharmaceutically acceptable.

Herein, the term "adjuvant" refers to an agent that increases the effect of the peptide of the invention when given jointly to it or forming part of the same treatment protocol. The pharmaceutically acceptable adjuvants and vehicles that may be used in the pharmaceutical composition of the present invention are vehicles known by those skilled in the art.

The composition of the invention may be employed as a therapeutic agent to be administered to a subject. In this case, the composition provided by this invention is considered a pharmaceutical composition which may be administered by any suitable administration route; to this end, said composition will be formulated in the suitable form for the selected administration route.

Routes of administration include topical, ocular, nasal, pulmonary, buccal, parenteral (intravenous, subcutaneous, and intramuscular), oral, vaginal and rectal. Administration from implants is also possible. The composition of the invention, in particular, the pharmaceutical composition, may also be administered topically to the skin or mucosa, that is, dermally or transdermally.

The composition of the invention may also be administered directly into the blood stream, into muscle, or into an internal organ. Suitable means for parenteral administration include intravenous, intra-arterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

The composition of the invention may also be administered intranasally or orally. Dosage forms for oral administration include, without limiting to, tablets (e.g., coated or uncoated tablets), capsules (e.g., soft gelatin capsules, hard gelatin capsules, HPMC capsules, or HPMCP capsules), a capsule inside a capsule, lozenges, troches, ovules, solutions, emulsions, suspensions, syrups, elixirs, powders and granules for reconstitution, dispersible powders and granules, medicated gums, chewing tablets, effervescent tablets, and multiparticulate dosage forms.

In a more preferred embodiment of the invention, the pharmaceutically acceptable atracurium salt, more preferably the cisatracurium besylate, or the composition comprising it, is administered orally, parenterally, intramuscularly, intra-arterially, intravenously, subcutaneously, epidurally, intradermally, intraperitoneally or intravesicularly.

In an even more preferred embodiment of the invention, the pharmaceutically acceptable atracurium salt, more preferably the cisatracurium besylate, or the composition comprising it, is administered intraperitoneally.

As understood by the person skilled in the art, the pharmaceutically acceptable atracurium salt, more preferably the cisatracurium besylate, have to be present in an effective amount, preferably in a therapeutically effective amount, in order to exert their effects.

In a preferred embodiment, the pharmaceutically acceptable atracurium salt, more preferably the cisatracurium besylate or the composition comprising it, comprises a therapeutically effective amount of the combination of pharmaceutically acceptable atracurium salt, more preferably cisatracurium besylate.

The skilled person in the art can determinate the pharmaceutically acceptable atracurium salt effective dosis. The effective amount may vary depending on, for example, the age, body weight, general health, sex and diet of the subject, as well as on the mode and time of administration, the excretion rate or any potential co-treatment with other drugs.

In a preferred embodiment, the dosis of the pharmaceutically acceptable atracurium salt is between 0.01mg per kg of subject (mg/kg) and 5mg/kg.

In a more preferred embodiment, the dosis of the pharmaceutically acceptable atracurium salt is between 0.05mg/kg and 1mg/kg, preferably is between 0.07mg/kg and 0.5mg/kg.

In an even more preferred embodiment, the dosis of the pharmaceutically acceptable atracurium salt is between 0.1mg/kg and 0.4mg/kg, preferably the dosis is 0.1 mg/kg, 0.15 mg/kg, 0.2 mg/kg, 0.25 mg/kg, 0.3 mg/kg, 0.35 mg/kg or 0.4 mg/kg.

In other aspect, the invention relates to the use the pharmaceutically acceptable atracurium salt above described, more preferably the cisatracurium besylate, or the composition comprising it, in the manufacture of a medicament, or in the manufacture of a pharmaceutical composition, for the treatment and/or prevention of a bone fragility-related disease, preferably osteogenesis imperfecta or osteoporosis, in a subject. Techniques and procedures for the production of medicaments are widely known in the state of the art.

The terms used to define the present aspect of the invention have been previously explained, and they and their preferred embodiments are applicable to the present aspect of the invention.

In another aspect, the invention relates to a method for the treatment and/or prevention of a bone fragility-related disease, preferably osteogenesis imperfecta or osteoporosis, comprising the administration of the pharmaceutically acceptable atracurium salt, more preferably the cisatracurium besylate, or the composition comprising it, to a subject.

In a preferred embodiment, the method further comprises a step, previous to the pharmaceutically acceptable atracurium salt, more preferably the cisatracurium besylate administration, of identifying a subject suffering from a bone fragility-related disease, preferably osteogenesis imperfecta or osteoporosis.

The terms used to define the present aspect of the invention have been previously explained, and they and their preferred embodiments are applicable to the present aspect of the invention.

### DESCRIPTION OF THE DRAWINGS

**Fig 1****.** Proliferation of OI-MSCs stained with DRAQ5. OI-MSCs were grown undifferentiated (UD) or under osteogenic differentiation (DIF) conditions, for 7 days and stained with DRAQ5. Each point represents a specific OI-MSCs cell line. Paired t test was done (** p< 0.01). UD n = 8; DIF n = 8. The experiment was repeated three independent times.
**Fig 2****.** Quantification of number of cells in the presence of G04 using DRAQ5. OI-MSCs were undifferentiated (UD) or under osteogenic differentiation (DIF) conditions, for 7 days. Number of cells was measured by DRAQ5 marker. Administration of 10µM of G04 was measured. Results were normalized to UD or DIF untreated conditions. Each point represents a specific OI-MSCs cell line. Paired t test was done (** p< 0.01). UD n = 6; DIF n = 6. The experiment was repeated three independent times.
**Fig 3****.** Study of apoptosis of OI-MSCs treated with 10 µM G04 by flow cytometry. Percentage of live and dead cells (early apoptotic, late apoptotic and necrotic) is represented. Five different conditions were studied. The percentage of live cells was compared in all the conditions. Paired t test was made (* p<0.05, **** p<0.0001).
**Fig 4****.** ALPL expression of OI-MSCs treated with G04 at day 7. MSCs were grown in normal (Day 7 UD) and osteogenic differentiation (Day 7 DIF) medium during 7 days. OI-MSCs were treated with 10 µM of G04. ALPL expression was normalized to GAPDH. Each point corresponds to a different OI-MSC line. Results are normalized to untreated conditions. Paired t test was made (* p<0.05, *** p<0.001). UD n = 8; DIF n = 4.
**Fig 5****.** Alizarin red staining of OI-MSCs treated with G04 molecule. For ARS quantification, absorbance was measured at 562 nm and normalized to protein content. Mineral deposition of OI-MSCs at day 12 and 15 of osteogenic differentiation was measured. OI-MSCs were treated with 10 µM of G04. Results are normalized to untreated conditions. Each point represents a specific OI-MSCs. Paired t test was done (*** p<0.001, ****p<0.0001). DIF day 12 n=10; DIF day 12 n=7.
**Fig 6****.** Immunofluorescence staining quantification of CHP of OI-MSCs treated with G04. MSCs are grown in normal (Day 12 UD) and osteogenic differentiation (Day 12 DIF) conditions, under G04 treatment (10 µM). Quantification of misfolded collagen is normalized to type I collagen. Each point represents a different OI-MSC line. Results are normalized to untreated condition. Paired t test was made (* p<0.05, ** p<0.01). UD n=5; DIF n=4.

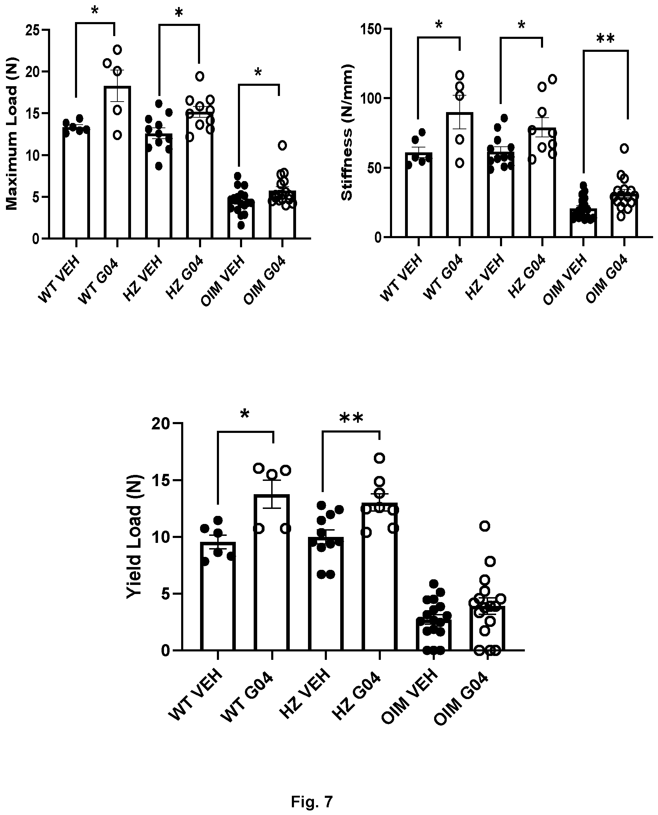

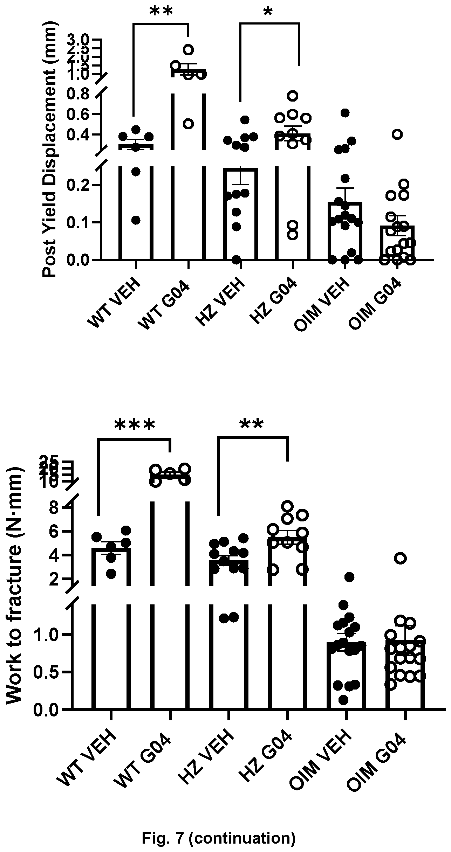

**Fig 7****.** Biomechanical study of male WT, HZ and OIM mice treated with G04 by three-point bending. Maximum load, stiffness, yield load, post-yield displacement and work to fracture were measured. Mice treated with vehicle and are represented. Each point corresponds to a different mouse. Unpaired t test was performed (* p<0.05, ** p< 0.01, *** p< 0.001). WT male vehicle n = 6; WT male G04 low n = 5; HZ male vehicle n = 11; HZ male G04 low n = 10; OIM male vehicle n = 15; OIM male G04 low n = 17.

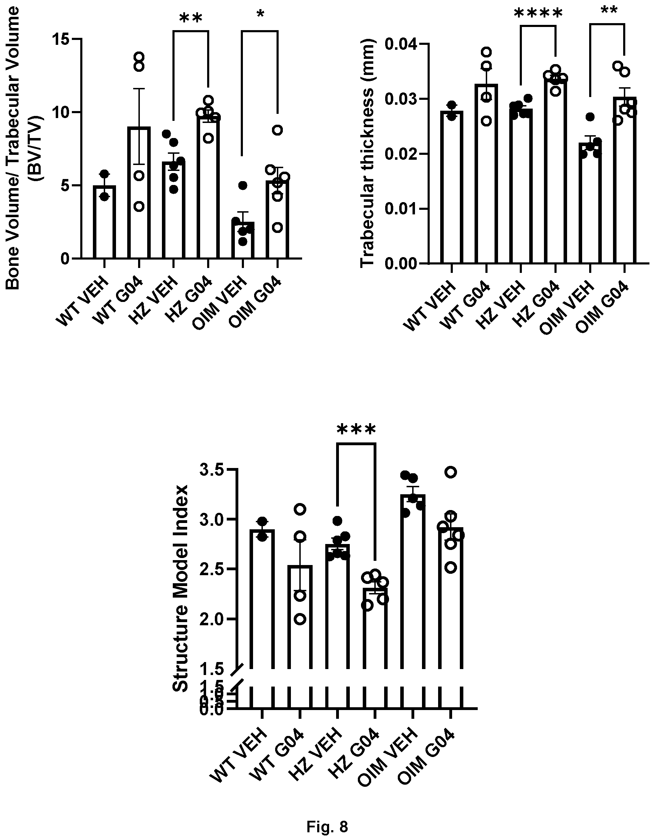

**Fig 8****.** Microstructure study of trabecular bone of male WT, HZ and OIM mice treated with low doses of G04 by µCT. Bone Volume/ Trabercular Volume (BV/TV), Trabercular thickness (Tb.Th) and Structure Model Index (SMI) were analyzed. Mice treated with vehicle and G04 are represented. Each point corresponds to a different mouse. Unpaired t test was done (* p<0.05, ** p< 0.01, *** p< 0.001, **** p< 0.0001). WT vehicle n = 2; WT G04 n = 4; HZ vehicle n = 6; HZ G04 n = 5; OIM vehicle n = 5; OIM G04 n = 5.

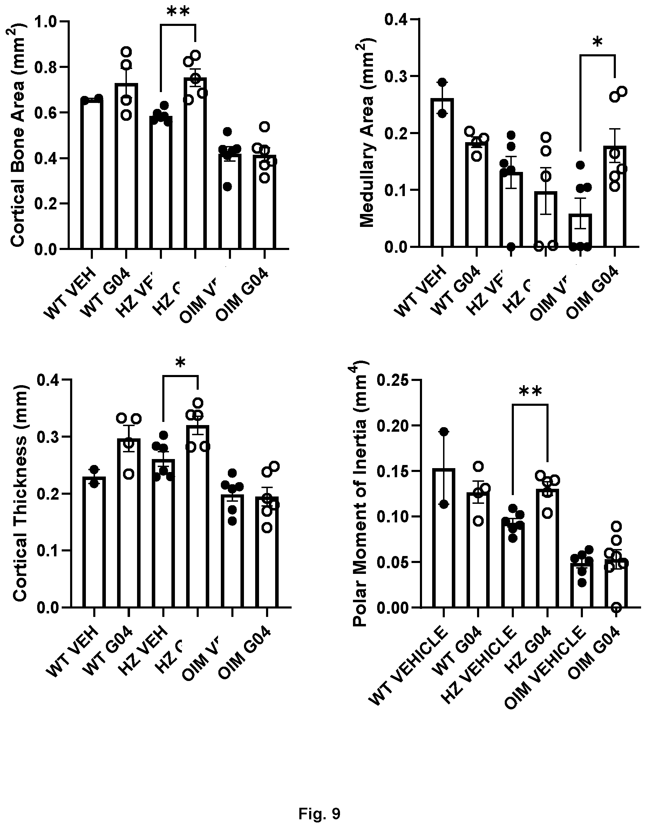

**Fig 9****.** Microstructure study of cortical bone of male WT, HZ and OIM mice treated with low doses of G04 by µCT. Cortical Bone Area (Ct.Ar), Medullary Area (Me.Ar), Cortical Thickness (Ct.Th) and polar moment of inertia (pMOI) were analyzed. Mice treated with vehicle and G04 are represented. Each point corresponds to a different mouse. Unpaired t test was done (* p<0.05, ** p< 0.01). WT vehicle n = 2; WT G04 n = 4; HZ vehicle n = 6; HZ G04 n = 5; OIM vehicle n = 6; OIM G04 n = 6.

### EXAMPLES

### MATERIALS AND METHODS

### Collection of human bone samples

The collection of human bone fragments and the study were approved by the Basque Ethics Committee for Clinical Research and conducted in accordance with the ethical standards formulated in the Helsinki Declaration. In some cases, pediatric patients with OI undergo surgical intervention to correct deformities and/or prevent fractures. During surgery, residual tiny bone pieces are sometimes discarded. The inventors subsequently collected those bone fragments in order to then obtain OI patient-derived MSCs (OI-MSCs). Bone fragments were donated from OI pediatric patients that present mutations in the COL1A1 or COL1A2 genes. In all cases informed consent was obtained.

### MSCs isolation and expansion

After donation, bone fragments were washed three times with Dulbecco's Phosphate Buffered Saline with CaCl₂ and MgCl2 (DPBS +/+) (Gibco, USA, REF: 14040-09) and then cut into small pieces. MSCs growth medium was added, composed by Dulbecco's Modified Eagle Medium (DMEM) (1X) with glutaMAX, low glucose (1g/L D-glucose) and pyruvate (Gibco, REF: 21885-025), 1% penicillin/streptomycin (Gibco, REF: 15140-122) and 10% fetal bovine serum (FBS) (Sigma-Aldrich, USA). First explant was left undisturbed for one week, until MSCs migrate to the cell culture plate. Next, bone pieces were transferred to a new plate and MSCs growth medium was added again. The plate from the first explant was washed with DPBS +/+ and filled with fresh media. After another week, cells should have migrated from bone to cell culture plate. Finally, MSCs obtained from first and second explants were expanded. Thus, cells were washed with Dulbecco's Phosphate Buffered Saline (DPBS -/-) (Gibco, REF: 14190-094) and detached with trypsin (TrypLE, Gibco, REF: 12563-011), incubating them at 37°C for 5 minutes. MSCs were collected and centrifuged at 300 x g during 5 minutes. Cells were resuspended in MSCs growth medium and counted using a Neubauer Chamber.

Cell concentration was calculated (cell number/ml) and cells were seeded at a range of 4000cells/cm². When the flasks reached a confluence of 80-90%, the process was repeated until a wide amount of cells was obtained. During the expansion process, cells that were not seeded were frozen (500000 cells/vial). Hence, MSCs medium was mixed with 10% dimethyl sulphoxide (DMSO) (Sigma-Aldrich, REF: D2650). Frozen cells were immediately stored at -80°C and after 24 hours, transferred to liquid nitrogen tank.

All the reagents used (DPBS, TrypLE^{™} and MSCs growth medium) were warmed to 37°C before use. Cells were grown in a cell culture incubator at constant 37°C and 5% CO₂ with saturating humidity to mimic endogenous conditions.

It is important to mention that during the first and second explants, 1ml of conditioned media was obtained from the culture in order to test the presence of Mycoplasma by a detection kit based on Polymerase Chain Reaction (PCR) (VenorGeM Classic, REF:11-1100). All the samples obtained were negative for Mycoplasma.

### MSCs characterization

It is essential to ensure that the cells obtained from bone explants were MSCs. For their correct characterization, rules proposed by the International Society for Cell and Gene Therapy were followed.

First, MSCs were plastic-adherent when maintained in standard culture conditions. Thus, after cell medium change, non-adherent cells are discarded. Second, MSCs express CD105, CD73 and CD90, and lack expression of CD45, CD34, CD14, CD19 and HLA-DR surface molecules. The expression of those markers was assessed by flow cytometry. Cells at passage 2-3 were detached and 1x10⁶ cells were used for the characterization. Cells were washed with 1 ml of DPBS +/+ with 2% of FBS and then blocked with blocking buffer (DPBS +/+ with 1% of Bovine Serum Albumin (BSA)(Fisher Scientific, BP1605-100)) during 1 hour at Room Temperature (RT). Then, cells were divided into 14 tubes and 2 ml of blocking buffer added. After centrifugation at 300 x g during 5 minutes, supernatant was discarded and cells were resuspended with 100 µl of blocking buffer. 3 µl of monoclonal antibody was added to each corresponding tube and incubated in darkness during 15 minutes at RT. Tubes were then washed with DPBS +/+. Finally, cells were resuspended in 300 µl of DPBS +/+ and analyzed in the cytometer (MACSQuant, Miltenyi Biotec). At least 200000 events were acquired for each antibody. Third, MSCs must differentiate to osteoblasts, adipocytes and chondroblasts in vitro. The differentiation to chondroblasts was not tested *in vitro* due to its complexity. On the contrary, the differentiation potential to osteogenic (all the cell lines) and adipogenic (some of the cell lines) lineages were tested by using specific cell culture media.

### MSCs culture and osteoblastic differentiation

OI-MSCs and CTRL-MSCs were used for *in vitro* experiments between passage 2 and 6. As they are primary cultures, higher passages are avoided because they showed alterations in morphology, response to stimuli, growth rates and protein expression. Both Ol- and CTRL-MSCs were cultured under MSCs growth medium. To induce osteogenic differentiation, cells were growth under MSCs osteogenic differentiation media: MSCs growth medium plus ascorbic acid 0.2 mM (Sigma-Aldrich, REF: A4403), β-glycerophosphate disodium salt hydrate 10 mM, (Sigma-Aldrich, REF: G9422) and dexamethasone 10 nM (Sigma-Aldrich, REF: G4902). Osteogenesis was always induced one day after cells were seeded (day 0). In all the cases the medium was changed at least twice a week.

### MSCs treatment in vitro

The effect of G04 (Cisatracurium besylate) in CTRL- and OI-MSCs was studied. MSCs without or under osteogenic differentiation were treated with G04 (FDA-approved molecule: Cisatracurium besylate, Sigma-Aldrich, REF: Y0001766). The molecule was added one day after the seeding (day 0) at a final concentration of 10µM.

### Apoptosis measurement

Apoptosis was measured by flow citometry based on the previously described protocol (Terrén, I., et al., 2023, IL-12/15/18-induced cell death and mitochondrial dynamics of human NK cells. Front Immunol, 14, 1211839). LIVE/DEAD and Apotracker dyes were used to separate live early apoptotic, late apoptotic and necrotic cells. OI-MSCs were seeded in 6-well plates at a confluence of 30000 cells/well. An additional well was seeded as negative control, for unstained cells. Next day, MSCs media was changed (growth media or osteogenic differentiation media) and 10 µM of G04 treatment was added to the selected wells. At day 5, media was changed, and a positive control of apoptosis was added: MSCs growth medium with 5% DMSO. At day 7, cells were collected (adherent cells together with death cells that are floating in the medium) and stained for flow cytometer analysis. 500000 MSCs were collected in flow cytometry tubes and washed twice with 2 mL DPBS-/-. MSCs were centrifuged at 1500 rpm during 5 minutes at room temperature and supernatant discarded.

Then, 50 µl of antibody staining mix was added per tube and incubated in darkness during 30 minutes at RT: 50 µl DPBS -/-, 0.2 µl APC-Cy7 Live/Dead Near IR (Thermo fisher, REF: L34994) and 0.5 µl (0.8 µM) FITC Apotracker Green (BioLegend, REF: 427403), a phosphatidylserine-binding fluorescent probe. Samples were washed with 2 ml DPBS -/-, centrifuged at 1500 rpm during 5 minutes at room temperature and supernatant was discarded. MSCs were manually resuspended and 200 µl of DPBS -/- added. Finally, data was acquired in the cytometer (MACSQuant, Miltenyi Biotec).

### Alizarin red staining

Alizarin Red S (ARS) forms a red-coloured complex with calcium ions, highlighting the areas of calcification within the cell culture, so it was used to stain the mineralized matrix of MSCs undergoing osteogenesis. OI-MSCs were seeded in a concentration of 15000 cells/well in 12 well plates. The following day, medium was change to osteogenic differentiation medium and treatment was added. As a negative control, MSCs growth medium was also added, in which no osteogenic differentiation is expected. Medium was changed twice a week. After 12 and 15 days of osteogenic differentiation, staining of the calcium deposits was performed. First, cells were washed with 1 ml of DPBS -/- and fixed with 300 µl/well of 4% paraformaldehyde for 30 min. Then, the cells were washed with 1 ml of distilled H2O and stained with 1 ml of 2% ARS solution (Thermo Fisher Scientific, REF: C.I 58005) at pH 4.1-4.3 for 45 minutes at RT in a shaker. After staining, MSCs were washed two times with 1 ml/well of distilled H2O. To quantify the mineralization, MSCs were destained for 15 minutes at RT in a shaker. For that, 500 µl/well of 10% cetylpyridinium chloride (SigmaAldrich, REF: C0732) in 0.1 M sodium phosphate buffer (prepared with Na2HPO4 (Panreac Quimica SLU, REF: 141679.1211) and NaH2PO4 (Panreac Quimica SLU, REF: 141965.1211)) were added. Afterwards, 100 µl of the liquid was transferred to a 96-well plate, and the absorbance was measured at 562 nm in a plate reader (iMark Microplate Reader, Bio-Rad). Each condition was seeded in triplicates in each plate. The experiment was repeated at least 2 times. It is important to note that under differentiation conditions, due to matrix deposition, MSCs generate a cell-sheet in vitro, which is really detachable when medium is changed. For that reason, some MSCs lines detached before day 12 or 15 of differentiation was reached. Thus, no mineralization results were available for those cell lines.

### Immunofluorescence

MSCs were seeded in chamber slides (Thermo Fisher Scientific, REF: 177437) (3000 cells/well) and cultured in MSCs growth medium. Next day, medium was changed to growth or osteogenic differentiation medium, and treatment was added (10 µM of G04). Medium was changed twice a week, and treatment added again. After 12 days of culture, the cells were washed with 500 µL of DPBS+/+ and fixed with 300µL of 4% paraformaldehyde for 10 minutes at room temperature. MSCs were washed three times with 500 µl of DPBS+/+ in a shaker for 5 minutes. Then, cells were permeabilized with 500 µl of permeabilization solution (0.2% Triton X-100 in DPBS+/+) for 30 minutes at RT in a shaker. Cells were washed once with 500 µl of DPBS +/+ and blocked with blocking buffer (3%BSA (Fisher Scientific, REF: BP1605-100) in 0.05% Triton X-100 in DPBS+/+) for 30 minutes at RT in a shaker. Next, cells were washed with 500 µl of DPBS+/+ and incubated with 20 µM heat-dissociated collagen hybridizing peptide, Cy3 conjugate (R-CHP; 3Helix, Salt Lake City, UT) and 3.33 µg/ml COL1 antibody (ab34710, Abcam; 1:300). For the preparation of the CHP before the incubation, it was heated at 80°C for 5 minutes and then cooled in ice for 1 minute. The antibody mix was diluted in 125 µl of DPBS+/+ and incubated overnight at 4°C in darkness. Next day, primary antibody was washed 3 times with 500 µl of DPBS+/+ for 5 minutes in a shaker. Cells were incubated with 125 µl of the secondary antibody (1:500) (Alexa Fluor 488 anti-rabbit, Invitrogen, REF: A11008) against rabbit, diluted in blocking buffer for 1 hour at RT in darkness. The secondary antibody was washed thrice with 500 µl of DPBS+/+ for 5 minutes, and once with 500 µl of distilled H₂O. OI-MSCs treated with 10 µM of G04 were stained with Hoechst (Thermo Scientific, REF: 62249) and the coverslips were mounted with mounting medium without DAPI (ProLong Diamond Antifade Mountant, Invitrogen, REF: P36965).

Finally, fluorescence images from 20 fields/well were scanned using a microscope "Zeiss Axio Observer.Z1 Apotome.2 in vivo". The objective used to acquire the images was LD A-Plan 10x/0.25 Ph1. The final optical resolution was 2.2026 pixels per micron. The exposure time was adjusted to avoid pixel saturation. To compare CHP and COL1 antibody signals, we calculated fluorescence staining values using ImageJ. The formula used for calculation was as follows: (red area × red fluorescence density)/(green area × green fluorescence density).

### RNA extraction, RT-PCR and qPCR

OI-MSCs and CTRL-MSCs were cultured (80000 cells/flask) in 25 cm² flasks under growth medium or ostegenic differentiation medium and then treated. After several days of differentiation and/or treatment, MSCs were detached following the previously mentioned protocol, and washed with 500 µL of DPBS +/+. Then, cells were centrifuged again at 300 x g during 5 minutes, supernatant was discarded and pellet was immediately frozen at -80°C. In the next step, total RNA was isolated from cells with the AllPrep DNA, RNA, MicroRNA extraction kit (QIAGEN, REF: 80224), following the manufacturers' provided protocol. Obtained RNA was quantified using the nanodrop (Nanodrop One, Thermo scientific), and checked that the 260/280 and 260/230 ratios were correct (1.8 -2.1 and 2.0-2.2, respectively). For mRNA expression analysis, 1µg of total RNA was reverse transcribed into complementary cDNA using the SuperScript IV VILO cDNA synthesis kit (Invitrogen, REF: 11756050) according to the manufacturer's instructions. The procedure was repeated 2 times for each line.

Finally, quantitative PCR (qPCR) was performed using 1:10 diluted cDNA. The reaction mix consisted of Brilliant III Ultrafast SYBR Green Q-PCR master mix (Agilent, REF: 600882), Forward and Reverse primers specific for each gene, 2 µL of each cDNA (1:10) and H₂O until 20 µL of reaction was reached. The primer concentration and the annealing temperature used are summarized in Table 1. The qPCR was performed in the MicroAmp^{™} Fast Optical 96-Well Reaction Plate (Applied biosystems, REF: 4346907) using the AriaMx RealTime PCR System (Agilent Technologies). Gene expression was normalized relative to glyceraldehyde-3-phosphate dehydrogenase (GAPDH) mRNA, and the relative expression of each gene of interest was calculated by the 2ΔCt method. Each point of the plate was done in triplicates. The quantification was repeated 2 times.

**Table 1. Primers used for cDNA quantification. Sequence, concentration and Annealing Temperature (Ann).**

| **Gene** | **Forward primer** | **Reverse primer** | **[Primer]** | **Ann** |
|---|---|---|---|---|
| *Col1a1* | GGGATTCCCTGGACCTAA AG (SEQ ID NO: 1) | GGAACACCTCGCTCTCCA (SEQ ID NO: 2) | 75nM | 59 |
| *Col1a2* | AGAGGACCACGTGGAGAA AG (SEQ ID NO: 3) | GGCCTGTGGGACCATCTT (SEQ ID NO: 4) | 75nM | 60 |
| *Alpl* | ACGAGCTGAACAGGAACA ACGT (SEQ ID NO: 5) | CACCAGCAAGAAGAAGCC TTTG (SEQ ID NO: 6) | 600nM | 58 |
| *CHRNA7* | TGGTGACAGTGATCGTGC TGCA (SEQ ID NO: 7) | GCCTCTTCATTCGCAGGA ACCA (SEQ ID NO: 8) | 75nM | 60 |
| *Gapdh* | CAAGGCTGAGAACGGAA GC (SEQ ID NO: 9) | AGGGGGCAGAGATGATGA CC (SEQ ID NO: 10) | 600nM | 58 |

### Murine model

All the in vivo experiments were carried out in the accredited facility Biobizkaia Health Research Institute. The studies were performed in accordance with the animal care and use protocol approved by this institution. The use of the OI model as well as the treatments administered, and all the protocols performed were designed following the 3R's principle. During this work, as a model of severe human OI, OIM mice maintained on the B6C3Fe-a/a-Col1a2 (OIM)/J background were purchased in the Jackson Laboratory. This same model in heterozygosis (+/OIM; HZ) was also used to resemble a moderate OI phenotype.

### Colony formation and mouse handle

All the mice were housed in the Biobizkaia Health Research Institute and had ad libitum access to water and food. To form the colonies, one male was coupled with two females in each cage. The best age of the males for the breeding is between 8 weeks and 9 months, and between 12-20 weeks for the females. Moreover, pregnancy of females lasts around 21 days. The colonies were formed in the following way. To obtain experimental mice, three different families were maintained in the case of OIM, in order to avoid consanguinity problems. Thus, first parental couple (F0) was always composed by a wild-type (WT) male and two HZ females. Heterozygous mice obtained in the second generation (F1) were only breeders and coupled with other HZ obtained from different families (HZ family 1 x HZ family 2). Finally, mice obtained from the HZ X HZ (F2) were assigned for experimental use: WT, HZ and OIM ones. Finally, in the case of OI murine models, due to the severe phenotype of OIM mice, it is imperative to handle them with care. Moreover, it is recommended to minimize their manipulation as much as possible to avoid fractures.

### Genotyping

Mouse genotyping was done by toe clipping at the age of 11 days. DNA was isolated from the small tissue, by Hot Sodium Hydroxide and Tris DNA isolation technique. First, 75 µl of alkaline lysis reagent (25mM NaOH (Sodium hydroxide, Sigma-Aldrich, REF: S8045), 0.2mM disodium EDTA (Ultrapure EDTA, Invitrogen, REF: 15576-028), pH 12) was added to each sample. Then, samples were heated to 95°C for 30 minutes in a thermoblock (thermos shaker TS100, Biosan) and cooled to 4°C on ice. Finally, the sample was neutralized with 75 µl of neutralizing reagent (40mM Tris-HCL, pH 5).

The same DNA extraction protocol was used for both OIM. Regarding the PCR, the mutation of the OIM mice was detected based on the following genotyping strategy: Two outer and two inner primers flanking the single nucleotide deletion were employed (Table 2). Those four primers were added to the same master mix with a final concentration of 0.5 µM, together with Kappa2G Fast HS genotyping Mix (x1) (Fisher Scientific, NC1645958) and 1 µl of DNA. The final volume per sample (25 µl) was reached with H₂O. The PCR program was as follows: 95°C for 3 minutes, then 30 cycles of 95°C for 15 seconds, 55.1°C for 10 seconds, and 72°C for 10 seconds, and lastly, 72°C for 1 minute. The product obtained was migrated in a 2% agarose gel (E-gel Double Comb 2% agarose with SYBR safe, Invitrogen, REF: A42348) for 13 minutes in the E-gel power snap (Invitrogen) with the E-gel double comb program. As a control, Fast Gene 100 bp DNA Ladder (Nippon Genetics, REF: MWD100) was used. After the migration, the products obtained were 440 bp (all genotypes), 303 bp (WT allele) and 195 bp (OIM allele) bands. After treatment and sacrifice, mice genotyping was repeated, by taking a small piece of tail and repeating the DNA extraction and PCR process.

**Table 2. Sequences of the primers employed for the genotype of oim.**

| **Mice** | **Primer name** | **Sequence** |
|---|---|---|
| OIM | Outer Forward | 5'-ACTGTCTGTCTACAGTGAACGTCTTAAT-3' (SEQ ID NO: 11) |
| | Outer Reverse | 5'-GATGTAGATGCATAGAAGACATGGAAGG-3' (SEQ ID NO: 12) |
| | Inner Forward (WT specific) | 5'-TTCCCATTTTTTTCTATTATACAGAAACAG-3' (SEQ ID NO: 13) |
| | Inner Reverse (OIM specific) | 5'-AATGATTGTCTTGCCCCATTCATTTTTT-3' (SEQ ID NO: 14) |

### Euthanasia and sample obtention

Mice were humanely euthanized according to the recommendations of the Guide for Care and Use of Laboratory Animals. Euthanasia was performed with CO₂ overdose, which causes rapid unconsciousness followed by death. Thus, mouse was placed in a small CO₂ cage and the CO₂ flow rate displaced 30% to 70% of the cage volume per minute. Mice are exposed to CO₂ until complete cessation of breathing was observed for a minimum of 2 minutes (normally, a total of 5-10 minutes may be required). The absence of movement and respiration must be visually confirmed. Relevant tissues such as blood, and long bones were harvested. Mice were first anesthetized with 4% of sevoflurane. Once the mouse was anesthetized, at the time of sacrifice, blood was collected by cardiac puncture with a 25G needle of 1 ml. In this case, a secondary physical method for euthanasia was used: exsanguination. Then, to obtain serum, blood was stored in a tube and let it clote for 30 minutes at room temperature. Finally, tubes were centrifuged at 1300 x g for 15 minutes, without brake. Serum aliquots were stored at -80°C. After blood obtaining, mice were dissected in order to collect hind limbs: femur and tibia. Bones were carefully extracted, cleaned and stored at -80°C for future analysis.

### Treatment with G04

All the treatments administered to OI mice were started when mice were young, at the age of 3 weeks. The treatment was administered intraperitoneally, three times a week, during one month. When mice reached seven weeks, they were euthanized and samples were collected. Stock solutions of the treatments was prepared in DMSO (Sigma-Aldrich, REF: D2650): 4 mg/ml of G04. Before each intraperitoneal injection, each mouse was weighted, and the treatment dose was adjusted accordingly. For that, stock was dissolved with physiological saline solution. Moreover, the DMSO tolerable dose in mice was taken into account. It is described that a dose higher than 30% of DMSO leads to severe clinical manifestations. Thus, in all the doses administered the amount of DMSO was never up to 6%. The final concentrations of the treatment was 0.8 mg/kg of G04. In parallel with the treatment, the same dose of vehicle (DMSO) was administered to control mice (termed as vehicle ones).

### Biomechanical testing: three-point bending

Biomechanical parameters of tibias were tested by three-point bending assay. The tibias treated with G04 were analyzed in Tecnun, School of Engineering (University of Navarra). Tibias were thawed at room temperature and subjected to three-point bending flexural tests. This test was performed attaching a three-point bend fixture to a universal uniaxial testing machine. The experimental setup consists of a Zwick/Roell machine model ZwickiLine Z1.0 (sn: 734188-2019). The load cell used was a 50 N Zwick/Roell Xforce P (sn: 781140). The software for controlling the machine and recording data was the Zwich/Roell testXpert III v1.4 with all patches installed up to the sx406-218-5. The tool used for performing the three-point bending test was homemade and consists of three stainless steel rolls (diameter 2 mm). Two of them are fixed to the lower part of the Zwickiline Z1.0. The axes of these two rods are 8 mm apart. The third rod is fixed in the crosshead of the machine, and it can move vertically. The axis of moving cylinder is parallel to the others. To compensate tool stiffness, a calibration cycle was performed in testXpert using an extremely stiff Widia bar (Widia THR-F 2.5 × 6.2 × 45.8 mm). This compensation guarantees that displacements recorded during the test correspond to real displacements applied on samples.

For performing biomechanical tests, bones were placed resting on the 2 lower rolls with condyles facing upwards. The positioning of bones was made manually with care to place the bone axis perpendicular to the rolls axis and as centered in the setup as possible. For each experiment, the registered force was zeroed before touching the bone with the upper roll. Once the bone was placed, the upper roll started moving downwards at 1 mm/min until the measured load reached 0.01 N. Once this preload was reached, the system started moving the upper roll downwards at 3 mm/min and recording the measured force and roll displacement. When the 90% of the maximum load decreases, the bone is considered to be broken, so the test is finished. All the parameters obtained from the force versus displacement plot were calculated with a Matlab program.

### Microarchitecture analysis: µCT

After dissection of long bones, the microarchitecture of treated tibias was analyzed by µCT in Washington University School of Medicine (St. Louis, MO, EEUU). Approximately a week before µCT, tibias were wrapped in gauze and stored in DPBS +/+ at - 20°C. For analysis, tibias were assembled in groups of five and embedded in 2% agarose. µCT analyses were conducted using the vivaCT 40 µCT scanner (SCANCO Medical AG, Bassersdorf, Switzerland) and the following parameters: 70kVp, 57 µA, 4 W X-ray energy intensity, high-resolution CT scan with a resolution of 7.4 µm, and an integration time of 700 ms296. For cortical analyses, 1 mm was contoured at the midshaft of each bone (135 slices) and for trabecular one, 1.5 mm were contoured starting at the growth plate (200 slices). Scans were analyzed using the SCANCO Medical µCT software system. All the contoured slices were analyzed for trabecular and cortical parameters and density thresholds set to 610 and 702 (lower), respectively, and 1000 (upper). After the µCT image analysis, the morphometric parameters calculated were the following: for trabecular bone, BV/TV, Tb. N, Tb.Th, Tb.Sp, BMD and SMI; and for cortical bone, T.Ar, Ct.Ar, Me.Ar, Ct.Th, TMD and pMOI.

### Statistical analysis

Statistical analysis was performed using GraphPad Prism. In the case of the *in vitro* experiments, when the same cell line was studied (with versus without treatment), paired test was used. In the case of *in vivo* experiments, unpaired test was used in all the cases, since each mouse is different. Then, regarding the distribution of the sample, normality tests were performed in all the cases before assuming a normal distribution. In general, Kolmogorov Smirnov test was made, but in the case that the sample was too small, Saphiro Wilk test was applied. Depending on the result of the normality test, different statistical analysis was performed. Under a normal distribution of the sample, t test was made (paired or unpaired). However, when it was assumed that the distribution of the sample was not normal, Wilcoxon (when paired) or Mann-Whitney U (in the case of unpaired) test was used. Quantitative data were obtained from separate experiments and calculated as mean ± standard error of the mean (SEM). A p value < 0.05 was considered significant.

### RESULTS

### Number of cells: DRAQ5

In order to evaluate the effect of 10 µM of G04 in 8 different lines of OI-MSCs, under UD and DIF conditions, cell number was measured with the DRAQ5 fluorescent probe, which stains DNA, by in-cell western technique. First the response to the differentiation stimuli of each OI-MSCs line was tested. An increase of the number of cells is expected under osteogenic differentiation conditions. All the DIF OI-MSCs lines studied, reflected a higher number of cells when comparing with the UD ones (Figure 1). In the OI-MSCs, a decrease on the number of cells was observed when treated with the concentration of 10 µM G04, in all the conditions studied, at day 7 (Figure 2).

### Apoptosis of OI-MSCs

In order to study if the treatment of 10 µM G04 induces apoptosis, nine OI-MSCs lines were evaluated by flow cytometry. Two different markers were used to divide live, early apoptotic, late apoptotic and death, necrotic cells. OI-MSCs were treated with 10 µM G04 in UD (Undifferentiated) and DIF (Differentiated) conditions, for 7 days. As a positive control, each OI-MSC line was treated with 5% DMSO. In all the cases, both detached (dead ones) and attached cells were studied together. As expected, the positive control with DMSO showed increased percentages of apoptotic cells. In conclusion, the reduction in the number of OI-MSCs treated with 10 µM G04 was not due to an increase in the apoptosis of the cells. Thus, it is due to a decreased proliferation (Figure 3).

### Early osteogenic differentiation: alkaline phosphatase

Early stages of osteogenic differentiation are generally studied with the alkaline phosphatase marker. Both undifferentiated (UD) and differentiated (DIF) OI-MSCs treated with 10 µM G04 exhibited decreased ALPL gene expression, as shown in Figure 4.

### Late osteogenic differentiation: mineralization

Late stages of osteogenic differentiation *in vitro* are a challenge to study, The most commonly used technique to study mineralization is alizarin red staining (ARS), which measures the calcium deposits secreted by the cells during differentiation. Under osteogenic differentiation conditions, ten OI-MSCs demonstrated an increase of calcium deposits (measured by ARS) at day 12 and 15. When differentiated OI-MSCs were treated with 10 µM G04, calcium content was decreased in the cell culture at day 12 and 15, when comparing with untreated controls (Figure 5).

### Misfolded collagen expression

Other interesting marker in OI is the amount of mutated collagen secreted by the cells. The use of CHP peptide gives us the chance to semi-quantify the amount of misfolded collagen. After 10 µM G04 treatment, the ratio of misfolded collagen/total collagen decreased, indicating less amount of mutant collagen after the treatment (Figure 6). The result was observed at day 12, both in UD and DIF conditions.

### Mice Treatment

For the *in vivo* studies, 3 weeks OIM mice (which model severe OI) were intraperitoneally treated with 0.8 mg/kg of G04. The treatment was administered 3 times a week during 1 month. As a control, mice were treated only with the vehicle (DMSO). After the treatment, right tibia was isolated and evaluated under 3 point bending test and different bone biomechanical parameters were measured. Between the parameters measured, the maximum load is indicative of the strength of the bone. The stiffness represents the bone's resistance to elastic deformation. The yield load measures how much load the bone can sustain before it suffers permanent damage. Post-yield displacement is a measure of ductility. Finally, work to fracture represents the energy that the bone can absorb prior to failure, and it is used to measure structural toughness. In this line, each point of the figure 7 represents a different mouse. Moreover, those mice treated with control vehicle are illustrated as full circles, whereas those treated with G04 are empty circles. For each parameter, WT, HZ (moderate OI) and OIM (severe OI) mice are represented.

When mice were treated with G04 molecule, elevated levels of the five studied parameters were observed in WT mice when compared to vehicles. These results indicate that G04 treatment increases bone strength, resistance to elastic deformation, ductility and toughness. In the case of HZ mice, an increase in maximum load, stiffness, yield load and work to fracture was reported when mice were treated with G04. In this case, HZ mice treated with the G04 molecule present increased bone strength, resistance to elastic deformation and tough. Finally, regarding OIM mice, maximum load and stiffness were higher, resulting in an increase of bone strength and resistance (Figure 7).

Regarding bone microarchitecture, tibia of G04 treated mice showed amelioration in some of the trabecular parameters studied (Figure 8). In this case, bone volume fraction (bone volume (BV)/total volume (TV)), Trabecular thickness (Tb.Th) and Structure model index (SMI) were evaluated. In fact, BV/TV ratio indicates the proportion of the total bone volume occupied by mineralized tissue. Moreover, Tb.Th measures the average thickness of individual trabecula. Finally, SMI, is related to the connectivity of the trabecular network. Here again, each point of the figure 8 represents a different mouse. Moreover, those mice treated with control vehicle are illustrated as full circles, whereas those treated with G04 are empty circles. For each parameter, WT, HZ (moderate OI) and OIM (severe OI) mice are represented. HZ mice demonstrated improved BV/TV, Tb.Th and SMI. These results demonstrated a higher bone volume occupied by mineralized tissue, thicker trabecula and improved trabecular network when HZ mice were treated with G04. Furthermore, OIM mice also demonstrated elevated BV/TV and Tb.Th in comparison to the control mice treated with vehicle, suggesting increased bone volume occupied by mineralized tissue and thicker trabecula, respectively.

With regards to cortical bone, different parameters were measured: Cortical bone area (Ct.Ar), Medullary area (Me.Ar), Cortical thickness (Ct.Th) and Polar moment of inertia (pMOI). In this case, Ct.Ar measures the area occupied by the hard, dense outer layer of the bone called cortical bone. Me.Ar indicates the area occupied by the medullary cavity, the hollow space in the centre of most bones. Then, Ct.Th is the measurement of the distance from the inner to the outer surface of the cortical bone layer. Finally, pMOI describes bone's resistance to bending forces. In the figure 9, each point represents a different mouse. Moreover, those mice treated with control vehicle are illustrated as full circles, whereas those treated with G04 are empty circles. For each parameter, WT, HZ (moderate OI) and OIM (severe OI) mice are represented. Subsequently, when HZ mice were treated with G04, an increase in Ct.Ar, Ct.Th and pMOI was observed in comparison to vehicles. Results suggest increased cortical area, thicker cortical structure and more resistant bone. Moreover, OIM mice exhibited increased Me.Ar when treated with G04, suggesting increased area of the medullary cavity (Figure 9).

In conclusion, these results indicate that G04 has an impact in different OI experimental models. On the one hand, it decreases the proliferation (but not the apoptosis), and early and late stages of osteogenic differentiation of OI patient-derived MSCs. Interestingly, misfolded collagen is a key factor of the altered phenotype that OI presents. G04 molecule has demonstrated the ability to decrease the amounts of misfolded collagen, suggesting a beneficial effect in OI-MSCs.

On the other hand, murine OI models (both with a moderate and a severe phenotype of the disease) treated with the molecule demonstrated increased bone strength and improved trabecular and cortical structure. These factors are essential to reduce the fragility of bones, the best-known characteristic of OI patients.

In accordance with the obtained results, since G04 treatment is able to improve bone strength and structure, it could be extrapolated to other diseases related with bone fragility. That is the case of osteoporosis, the most common chronic metabolic bone disease.

## Claims

1. A pharmaceutically acceptable atracurium salt for use in the treatment and/or prevention of a bone fragility-related disease in a subject.

2. The atracurium salt for use according to claim 1, wherein the bone fragility-related disease is osteogenesis imperfecta or osteoporosis.

3. The atracurium salt for use according to claim 1 or 2, wherein the salt is from mesylate or besylate.

4. The atracurium salt for use according to any of the claims 1 to 3, wherein the salt is from besylate.

5. The atracurium salt for use according to any of the claims 1 to 4, wherein the atracurium is cisatracurium.

6. The atracurium salt for use according to claim 5, wherein the cisatracurium salt is cisatracurium besylate.

7. A pharmaceutical composition comprising a pharmaceutically acceptable atracurium salt defined in any of the claims 1 to 6, for use in the treatment and/or prevention of a bone fragility-related disease in a subject.

8. The composition for use according to claim 7, wherein the bone fragility-related disease is osteogenesis imperfecta or osteoporosis.

9. The composition for use according to claim 7 or 8, wherein the pharmaceutical composition comprises a pharmaceutically acceptable carrier, an excipient and/or an adjuvant.

10. The atracurium salt or the pharmaceutical composition for use according to any of the claims 1 to 9, wherein the subject is a human, preferably a human under 18 years of age.

11. The atracurium salt or the pharmaceutical composition for use according to claim 10, wherein the human is a male.

12. The atracurium salt or the pharmaceutical composition for use according to any of the claims 1 to 11, wherein the atracurium salt is cisatracurium besylate and the dosis of cisatracurium besylate is between 0.05mg/kg and 1mg/kg of subject.

13. The atracurium salt or the pharmaceutical composition for use according to any of the claims 1 to 12, wherein the atracurium salt is administered orally, parenterally, intramuscularly, intra-arterially, intravenously, subcutaneously, epidurally, intraperitoneally or intravesicularly.

14. The atracurium salt or the pharmaceutical composition for use according to claim 13, wherein the atracurium salt is administered intraperitoneally.
